# EUROPÄISCHE PATENTSCHRIFT

(11) **EP 2 867 354 B1**
(45) Veröffentlichungstag und Bekanntmachung des Hinweises auf die Patenterteilung: **20.07.2016**
(21) Anmeldenummer: 13739139.7
(22) Anmeldetag: 19.06.2013
(51) Int. Cl.: C12M 1/107, C12M 1/00, C12M 1/12

(54) **ENERGIEVERSORGUNGSEINHEIT**
ENERGY SUPPLY UNIT
UNITÉ D'ALIMENTATION EN ÉNERGIE

(30) Priorität: 28.06.2012 DE 102012105658
(43) Veröffentlichungstag der Anmeldung: 06.05.2015
(73) Patentinhaber: Microbenergy GmbH, 92421 Schwandorf (DE)
(72) Erfinder: SCHMACK, Ulrich, 93059 Regensburg (DE); SCHMACK, Doris, 93138 Lappersdorf (DE)
(74) Vertreter: Glück Kritzenberger Patentanwälte PartGmbB
(86) Internationale Anmeldenummer: PCT/DE2013/100222
(87) Internationale Veröffentlichungsnummer: WO 2014/000731

(56) Entgegenhaltungen:
- WO-A1-2010/115983
- CN-A- 102 344 885
- CN-A- 102 344 886
- CN-U- 201 785 390
- JP-A- 06 169 783

## Beschreibung

### Technisches Gebiet

Die Erfindung betrifft eine Energieversorgungseinheit.

### Stand der Technik

Der Einsatz erneuerbarer Energien zur Stromerzeugung gewinnt aktuell immer mehr an Bedeutung. Insbesondere die Energiewende weg von fossilen Energieträgern und Kernenergie hin zu regenerativen Energiequellen fordert einen verstärkten Einsatz von erneuerbaren Energiequellen zur Stromerzeugung wie beispielsweise Windkraft, Solarenergie, Wasserkraft oder Geothermie.

Insbesondere im Fall von Windkraft und Solarenergie kommt es zu ausgeprägten witterungsbedingten sowie tages- und jahreszeitlich bedingten Schwankungen bei der Stromerzeugung. Dies führt mittlerweile dazu, dass bei starkem Windaufkommen Windkraftanlagen abgeschaltet werden müssen, um eine Überlastung der Stromnetze zu verhindern. Ähnliches gilt für die Stromüberproduktion aus Photovoltaik in Zeiten mit starker Sonneneinstrahlung und gleichzeitiger Nachfragelücke. Es wird also wertvolle Kapazität aus erneuerbaren Energiequellen nicht genutzt. Auf der Stromangebotsseite könnte billiger Überschussstrom in den Zeiten zur Verfügung gestellt werden, in denen nur eine geringe Nachfrage bei den Stromverbrauchern vorhanden ist.

Auf der Stromnachfrageseite kommt es ebenfalls zu starken Schwankungen. Beispielsweise fällt bei der Stromnachfrage eine Spitzenlast vor allem um die Vormittags- und Mittagszeit an. Auch in Kälte- oder extremen Hitzeperioden steigt die Stromnachfrage stark an. Strom, der außerhalb dieser Nachfragepeaks produziert wird, kann zu günstigeren Preisen erworben werden ("Peak-off"-Strom). Da sowohl die Stromerzeugung aus regenerativen Energiequellen wie auch der nachfrageabhängige Stromverbrauch stark schwanken, die Stromenergie im Stromnetz kaum gespeichert werden kann, jedoch jederzeit ein genügend großes Stromangebot vorhanden sein muss, um die Netzstabilität zu gewährleisten, besteht ein starkes Bedürfnis das Ungleichgewicht von fluktuierender Stromerzeugung durch regenerative Energien und der nicht korrelierenden, ebenfalls stark schwankenden Stromnachfrage auf Seiten der Verbraucher zu verbessern.

Ein Lösungsansatz zu dieser Problematik besteht darin, die elektrische Energie aus erneuerbaren Energiequellen in chemische Energie wie beispielsweise Wasserstoff oder Methan umzuwandeln und dann entweder direkt zu verwenden oder zwischenzuspeichern und anschließend wieder in elektrische Energie zurückzuverwandeln. Die Bildung von Methan aus Wasserstoff und Kohlendioxid erfolgt technisch in erster Linie durch ein physikalisch-technisches Verfahren (Sabatier Prozess) oder alternativ durch die biologische Umsetzung von H₂ und CO₂ mit Hilfe von Methan bildenden Bakterien.

In diesem Zusammenhang offenbart die DE 10 2007 037 672 A1 ein Verfahren zur nachhaltigen Energieversorgung mit einem Kohlenstoffkreislauf unter Einsatz von regenerativ erzeugtem Wasserstoff. Regenerativer Wasserstoff wird mit Hilfe von Elektrolyse unter Einsatz von Windstrom oder Solarstrom erzeugt. Dieser regenerative Wasserstoff wird zusammen mit Kohlendioxid, das aus Kraftwerksabgasen stammt, in einem modifizierten Fischer-Tropsch-Verfahren in einer Hydrieranlage in flüssige oder gasförmige Kohlenwasserstoffe umgewandelt, die entweder in Kraftwerken verbrannt und verstromt oder als Kraftstoffe für Fahrzeuge eingesetzt werden.

In der WO 2010/115938 A1 ist ein Energieversorgungssystem dargestellt, das regenerativ erzeugten Strom aus Wind- oder Sonnenenergie teilweise über Elektrolyse in Wasserstoff umwandelt, der dann zusammen mit Kohlendioxid in einer Methanisierungsstufe zu Methan umgesetzt wird. Das Kohlendioxid stammt entweder aus Kraftwerksabgasen, aus der Luft oder auch aus Biomasse. So kann abgetrenntes CO₂ aus einer Biogasanlage oder CO₂-haltiges Synthesegas aus der Biomassevergasung verwendet werden. Das synthetisch erzeugte methanhaltige Gas wird über eine Gasbereitstellungseinrichtung in Form eines Zusatz- oder Austauschgases in ein Gasversorgungsnetz eingespeist.

Die US 4,883,753 beschreibt eine Methanproduktion mit hoher Ausbeute in einem kontinuierlichen Kultursystem als Bioreaktor, in dem thermophile Methanbakterien der Art *Methanobacterium thermoautotrophicum* eingesetzt werden. Eine hohe Methanausbeute wird dadurch erreicht, dass ein hoher Gaseintrag an H₂ und CO₂ verwendet wird, wobei die Gasübertrittrate durch sehr hohe Rührgeschwindigkeiten im Fermenter unterstützt wird.

Die WO 2008/094282 A1 beschreibt ein biologisches System zur Methanproduktion aus Wasserstoff und Kohlendioxid unter Verwendung einer Bakterienkultur, die mindestens eine Art von methanogenen Bakterien enthält. Das Kohlendioxid kommt dabei aus einem industriellen Prozess, während der Wasserstoff u.a. durch Elektrolyse unter Einsatz von billigem Überschussstrom gewonnen werden kann. Höhere Methanausbeuten werden durch hohe Temperatur, hohe Rührgeschwindigkeiten, einen hohen Gaseintrag oder spezielle Reaktortypen wie geschichtete oder kaskadierende Reaktoren erreicht.

Die WO 2011/003081 A1 beschreibt ein System zur Erzeugung von Methan in einem biologischen Reaktor mit zwei Kammern, in dem bei hoher Temperatur direkt in dem Bioreaktor Wasserstoff durch Elektrolyse in der Kathodenkammer in Anwesenheit methanogener Mikroorganismen gebildet wird. Zur Methanerzeugung wird Kohlendioxid von außen in die Kathodenkammer zugeführt.

Die WO 2011/000084 A1 offenbart ebenfalls ein System zur Produktion eines methanreichen Gases, bei dem die Elektrolyse von Wasser unter Bildung von Wasserstoff und Sauerstoff direkt in einem Bioreaktor durchgeführt wird, der hydrogenotrophe methanogene Mikroorganismen enthält. Anode und Kathode sind hierbei nicht durch eine Membran getrennt. Das Kohlendioxid stammt vorzugsweise aus dem Bioreaktor selbst.

Die JPH06169783 A beschreibt ein System und eine Methode zur Erzeugung von Methan aus Wasserstoff und Kohlendioxid in einem biologischen Reaktor mit der Hilfe von methanogenen Bakterien. Wasserstoff wird durch Elektrolyse unter Verwendung von Solarenergie hergestellt.

Trotz dieser Lösungsansätze ist es bisher nicht geglückt, das Ungleichgewicht von fluktuierender Stromerzeugung durch regenerative Energien und stark schwankender Stromnachfrage signifikant zu mindern. Es besteht daher ein Bedarf an Energieversorgungsanlagen, die aus regenerativen Energieträgern gewonnene Energie ohne starke Leistungsschwankungen zur Verfügung stellen.

### Darstellung der Erfindung

Der Erfindung, wie sie in den Ansprüchen gekennzeichnet ist, liegt die Aufgabe zu Grunde, eine Energieversorgungseinheit bereitzustellen, die aus regenerativen Energieträgern gewonnene Energie ohne starke Leistungsschwankungen zur Verfügung stellt.

Diese Aufgabe wird erfindungsgemäß durch die Energieversorgungseinheit gemäß Anspruch 1 gelöst. Weitere vorteilhafte Details, Aspekte und Ausgestaltungen der vorliegenden Erfindung ergeben sich aus den abhängigen Ansprüchen, der Beschreibung und den Beispielen.

Die vorliegende Erfindung stellt eine Energieversorgungseinheit aufweisend eine Windkraftanlage zur Erzeugung von elektrischem Strom aus Wind, eine Elektrolysevorrichtung zur Erzeugung von Wasserstoff und Sauerstoff aus Wasser unter Verwendung des von der Windkraftanlage erzeugten elektrischen Stroms, einen Bioreaktor zur Herstellung von Methan aus Wasserstoff und Kohlendioxid unter Einsatz von methanogenen Bakterien, eine Zufuhrvorrichtung für die Zufuhr von Kohlendioxid in den Bioreaktor und einen in dem Bioreaktor vorgesehenen Auslass für das im Bioreaktor entstehende biomethanhaltige Gas zur Verfügung. Erfindungsgemäß ist der Bioreaktor in den Turm der Windkraftanlage eingebaut, wobei der Bioreaktor eine Höhe von mehr als 5 m aufweist.

Die erfindungsgemäße Energieversorgungseinheit kann die wegen Schwankungen in der Stromproduktion sowie der Stromnachfrage bei erneuerbaren Energien nicht genutzten Stromüberkapazitäten zu Zeiten, in denen billiger Überschussstrom auf dem Markt vorhanden ist, effizient nutzen, um methanreiches Gas aus Wasserstoff und Kohlendioxid zu bilden, welches dann als chemischer Energiespeicher oder Zwischenspeicher dient. Die vorliegende Erfindung zeigt einen Weg auf, aus erneuerbaren Energieträgern gewonnenen Strom über die Zwischenstufe einer Umwandlung in Wasserstoff und unter Zufuhr von Kohlendioxid in einem Bioreaktor unter Einsatz von methanogenen Bakterien zur Bildung von Methan zu nutzen, um so eine effizientere Nutzung von fluktuierend anfallendem, aus erneuerbaren Energieträgern gewonnenen Strom, insbesondere aus Windkraft, zu erreichen. Die biologische Methanisierung aus regenerativ erzeugtem Wasserstoff und Kohlendioxid erfolgt direkt am Ort der Windkrafterzeugung, nämlich innerhalb des Turmes der Windkraftanlage.

Bei der erfindungsgemäßen Energieversorgungseinheit ist ein biologischer Methanisierungsreaktor in den Turm der Windkraftanlage integriert. Ein derartiges System bringt gegenüber dem Stand der Technik verschiedene Vorteile mit sich. Ein technisches Problem bei der biologischen Methanbildung ist der mangelhafte Gaszu-Flüssigkeitstransfer, insbesondere für Wasserstoff. Der zugegebene gasförmige Wasserstoff soll sich möglichst vollständig im Medium lösen, sodass die Methan bildenden Bakterien den zugeführten Wasserstoff mit Kohlendioxid zu Methan umsetzen können.

In den Turm einer Windkraftanlage, der ohnehin bereits als Infrastruktur vorhanden ist, können mit relativ geringem Aufwand hohe und schlanke Bioreaktoren eingebaut werden. Die langen Reaktionsstrecken, die sich aufgrund der großen Reaktorhöhe ergeben, bewirken, dass sich der aufsteigende Wasserstoff besser im Medium lösen kann bzw. die Bakterien über eine größere Strecke Zeit haben, den Wasserstoff aufzunehmen und zu Methan umzusetzen. Zusätzlich ergibt sich aufgrund der hohen Wassersäule ohne weiteren Energieaufwand ein erhöhter Druck im Bioreaktor, was ebenfalls die Löslichkeit der zugegebenen Gase erhöht. Da die Elektrolyseeinrichtung direkt am Ort der Windkraftanlage installiert ist, kann überschüssiger Strom direkt vor Ort genutzt und in Form von chemischer Energie zwischengespeichert werden, ohne das Stromnetz zu überlasten.

Die als Bestandteil der erfindungsgemäßen Energieversorgungseinheit vorgesehene Windkraftanlage besitzt alle Komponenten, die aus dem Stand der Technik bekannt sind, um elektrischen Strom aus Wind zu erzeugen, insbesondere Rotorblätter, Nabe, Turm, Fundament, Aufstieg, Getriebe, Blattverstellung, Bremse, Gondel, Windrichtungsnachführung, Messinstrumente, Drehstromgenerator, Gleichrichter, Wechselrichter und Netzanschluss.

Als Synonyme für Windkraftanlage werden im Rahmen des vorliegenden Textes die Bezeichnungen Windkraftwerk, Windenergieanlage, Windkraftkonverter, Windrad oder Windgenerator verwendet.

Die Windkraftanlage kann als einzelnes Windrad ausgebildet sein oder sich in einem Windpark befinden. Die Windkraftanlage kann sich auf dem Festland (onshore) oder auf dem Meer (off-shore) befinden.

Der Turm der Windkraftanlage weist einen Hohlraum auf, in dem der Bioreaktor zur Methanbildung eingebaut vorliegt. Türme von Windkraftanlagen sind bevorzugt aus Stahl oder Stahlbeton gefertigt oder es handelt sich um Hybridkonstruktionen aus Stahl und Stahlbeton. Daneben existieren sogenannte Sandwichtürme mit einer Stahlinnen- und -außenhülle, die durch pumpfähige Elastomere oder Stahlbeton verbunden sind.

Gemäß einer bevorzugten Ausführungsform der vorliegenden Erfindung sind die Türme im Turminneren mit einer Leiter oder einem Aufzug und besonders bevorzugt zusätzlich mit einer Materialwinde ausgestattet. Über die Hälfte der 2011 installierten Windkraftanlagen wiesen eine Nabenhöhe von mehr als 100 m auf, 20% sogar eine Nabenhöhe von mehr als 120 m. Aktuell geplante Anlagen weisen eine Höhe von bis zu 200 m auf. Insbesondere diese neuerdings vermehrt installierten zunehmend höheren Windkraftanlagen weisen daher Turmhöhen auf, die geeignet sind, um Bioreaktoren mit einer Höhe bis über 100 m aufzunehmen. Ein im Turminneren gelegener Aufstieg oder Aufzug sowie eine Materialwinde können gleichzeitig als Hilfen für den Betrieb oder die Wartung des Bioreaktors dienen.

Bevorzugt weist die Windkraftanlage einen Netzregler auf, der den entstehenden Windkraftstrom entweder bei Bedarf ins elektrische Netz einspeist oder den Strom für eine anderweitige Anwendung freigibt. Bevorzugt wird der Überschussstrom aus der Windkraftanlage nicht nur zur Erzeugung von Wasserstoff unter Verwendung einer Elektrolyseeinheit verwendet, sondern auch für weitere stromverbrauchende Komponenten innerhalb der Energieversorgungseinheit wie den Betrieb des Bioreaktors oder der Anlage zur CO₂-Bereitstellung eingesetzt.

Über den gemäß einer bevorzugten Ausführungsform vorgesehenen Netzregler kann die Energieversorgungseinheit auch günstigen Überschussstrom nutzen, der nicht von der Windkraftanlage vor Ort, sondern anderswo im Netz produziert wird, beispielsweise bei Vorhandensein eines Stromüberangebots im Netz aus Photovoltaikanlagen bei starker Sonneneinstrahlung. Die Energieversorgungseinheit ist so in der Lage, am Regelenergiemarkt teilzunehmen und gegebenenfalls negative Regelenergie bereitzustellen, die durch die Energieversorgungseinheit verbraucht wird.

Erfindungsgemäß ist in dem Turm der Windkraftanlage ein Bioreaktor integriert, mit dessen Hilfe unter Einsatz von methanogenen Bakterien aus Wasserstoff und Kohlendioxid methanhaltiges Gas herstellt werden kann. Der Bioreaktor weist bevorzugt eine Zylinderform auf, wobei die Höhe des Reaktors seinen Durchmesser übertrifft. Bevorzugt beträgt das Verhältnis von Höhe zu Durchmesser mehr als 5 zu 1, besonders bevorzugt mehr als 10 zu 1. Der Bioreaktor weist erfindungsgemäß eine Höhe von über 5 m, bevorzugt eine Höhe von über 10 m, besonders bevorzugt eine Höhe von über 20 m auf. Der Bioreaktor ist bevorzugt aus Glas, Kunststoff oder Metall, insbesondere Edelstahl gefertigt.

Als methanbildende Mikroorganismen enthält der Bioreaktor mindestens ein geeignetes hydrogenotrophes methanbildendes Bakterium, das aus Wasserstoff und Kohlendioxid Methan bilden kann. Geeignete hydrogenotrophe methanbildende Bakterien sind alle im Stand der Technik einschlägig bekannten methanbildenden Bakterien aus dem Reich der Archaeen, insbesondere aus den Ordnungen *Methanobacteriales, Methanomicrobiales, Methanococcales, Methanopyrales, Methanocellales* oder *Methanosarcinales.* Geeignet sind sowohl mesophile Vertreter von methanogenen Bakterien (Temperaturoptimum im Bereich 20 °C bis 45 °C) als auch thermophile Vertreter (Temperaturoptimum im Bereich höher als 45 °C). In einer bevorzugten Ausführungsform werden Bakterien der Arten *Methanothermobacter thermautotrophicus, Methanothermobacter marburgensis, Methanothermobacter wolfeii, Methanococcus maripaludis, Methanococcus aeolicus, Methanococcus thermolithotrophicus* oder *Methanobacterium formicicum* verwendet. Es können sowohl Reinkulturen eines hydrogenotrophen methanbildenden Bakteriums als auch Mischkulturen von mehreren hydrogenotrophen methanbildenden Bakterien verwendet werden.

Zusätzlich zu mindestens einem hydrogenotrophen methanbildenden Bakterium können weitere methanbildende Bakterien wie acetoclastische Methanbakterien (z.B. *Methanosarcina sp., Methanosaeta sp.)* oder weitere geeignete Bakterien wie hydrolytisch wirkende Bakterien (z.B. *Clostridium sp., Acetobacterium sp., Ruminobacterium sp.)* oder elektrochemisch wirksame Bakterien (z.B. *Geobacter sp., Shewanella sp.)* die Kultur in dem methanogenen Bioreaktor ergänzen, so dass eine Mischkultur aus verschiedenen Bakterien eingesetzt wird.

Die Bakterien können frei in Lösung in dem Bioreaktor vorkommen oder immobilisiert an geeignete Trägerstoffe vorliegen.

Je nach Zusammensetzung der Bakterienkultur in dem Bioreaktor werden aus dem Stand der Technik bekannte Kulturmedien verwendet. Der Bioreaktor kann mit einer kontinuierlichen oder einer diskontinuierlichen Kultur betrieben werden. Bevorzugt besitzt der Bioreaktor eine Zufuhr- bzw. Abfuhreinrichtung für ein Kulturmedium. In einer Ausführungsform kann das Medium auch rezirkuliert werden, so dass beispielsweise in der Nähe des Reaktorbodens die Medienzufuhr erfolgt und im oberen Bereich des Bioreaktors die Medienabfuhr.

In einer bevorzugten Ausführungsform wird der Bioreaktor temperiert, so dass die Bakterien optimale Wachstumsbedingungen vorfinden. Eine Fermenterheizung kann sowohl im Reaktorinneren, bevorzugt in Form von Heizschlangen oder auch von außen, bevorzugt in Form einer Heizmanschette, installiert werden. Die Fermenterheizung kann mit Strom betrieben werden, der durch die Windkraftanlage erzeugt wird, insbesondere durch billigen Überschussstrom, oder auch über einen Wärmetauscher durch Abwärme, die durch einen anderen Prozess im Umfeld der Energieversorgungseinheit erzeugt wird. Insbesondere kann die Abwärme der Elektrolysevorrichtung zur Heizung des Bioreaktors genutzt werden. Unter bestimmten Bedingungen kann es auch notwendig sein, den Bioreaktor zu kühlen statt zu heizen, um eine zu starke Erhitzung der Mikroorganismen zu vermeiden. Die Temperierung des Bioreaktors kann daher in Form einer Temperaturerhöhung ebenso wie in Form einer Temperaturerniedrigung erfolgen. Eine Kühlvorrichtung kann ebenfalls mit billigem Überschussstrom aus der Windkraftanlage betrieben werden.

Der Bioreaktor besitzt eine Zufuhreinrichtung für die Zufuhr von Kohlendioxid. In einer bevorzugten Ausführungsform wird gasförmiges Kohlendioxid über eine ventilgesteuerte Leitung in Bodennähe des Bioreaktors zugeführt.

Die Elektrolysevorrichtung zur Erzeugung von Wasserstoff und Sauerstoff aus Wasser unter Verwendung zumindest eines Teils des von der Windkraftanlage erzeugten elektrischen Stroms kann außerhalb oder innerhalb des Bioreaktors angeordnet sein. Für den Fall, dass die Elektrolysevorrichtung innerhalb des Bioreaktors angeordnet ist, sind keine weiteren Vorkehrungen erforderlich, da der produzierte Wasserstoff direkt zur Methanisierung zur Verfügung steht. Ist die Elektrolysevorrichtung dagegen außerhalb des Bioreaktors angeordnet, so ist für den Fachmann klar, dass der Bioreaktor mit einer entsprechenden Zufuhreinrichtung für die Zufuhr des in der Elektrolysevorrichtung gebildeten gasförmigem Wasserstoffs ausgestattet ist. In einer bevorzugten Ausführungsform wird der gasförmige Wasserstoff über eine ventilgesteuerte Leitung in Bodennähe des Bioreaktors zugeführt.

Wasserstoff und Kohlendioxid können über zwei getrennte Gaseinlässe in den Bioreaktor eingebracht werden oder sie werden vor Eintritt in den Bioreaktor gemischt und über einen gemeinsamen Gaseinlass in den Bioreaktor eingebracht.

In einer weiter bevorzugten Ausführungsform werden die zugeführten Gase, insbesondere der Wasserstoff möglichst fein verteilt zugeführt, beispielsweise über entsprechende Fritten, feinporige Verteilerplatten, netzartige. Verteilergewebe, Membranen oder entsprechende Gaslanzen.

In unmittelbarer Nähe des Gaseinlasses kann ein Rührer installiert sein, der sowohl das Kulturmedium als auch die zugeführten Gase Wasserstoff und Kohlendioxid durchmischt und verteilt.

Der Bioreaktor der erfindungsgemäßen Energieversorgungseinheit besitzt einen Auslass für das im Bioreaktor entstehende biomethanhaltige Gas. In einer bevorzugten Ausführungsform liegt dieser Auslass im oberen Bereich des Bioreaktors, insbesondere am höchsten Punkt des Bioreaktors.

Das im Bioreaktor gebildete Gas besteht zu einem signifikanten Anteil aus Biomethan, das von hydrogenotrophen methanbildenden Bakterien aus H₂ und CO₂ erzeugt wird. Bevorzugt hat das im Bioreaktor gebildete Gas einen Biomethangehalt von mehr als 10 %, besonders bevorzugt einen Biomethangehalt von mehr als 25 %, insbesondere bevorzugt einen Biomethangehalt von mehr als 50 %. Neben Biomethan kann das entstehende Gas auch Anteile von H₂ und CO₂ sowie kleinere Konzentrationen an beispielsweise H₂S, O₂, N₂ und Wasser enthalten. Da das entstehende Gas zu einem signifikanten Anteil aus Biomethan besteht, wird es auch als biomethanhaltiges Gas bezeichnet.

In einer bevorzugten Ausführungsform wird das entstehende biomethanhaltige Gas ausgehend von dem Gasauslass in den Bioreaktor rezirkuliert. Besonders bevorzugt ist es, das biomethanhaltige Gas im oberen Teil des Bioreaktors zu entnehmen und am unteren Ende des Bioreaktors rückzuführen. Eine Rezirkulation des biomethanhaltigen Gases kann dazu dienen, die Qualität des Gases zu verbessern, insbesondere den Biomethangehalt durch nochmaliges Durchlaufen der Fermenterstrecke zu erhöhen.

Mit "Biomethan" wird Methan biologischen Ursprungs bezeichnet, das durch ein Verfahren zur biologischen Methanisierung durch methanogene Mikroorganismen in einem Bioreaktor aus zugeführtem Wasserstoff und Kohlendioxid erzeugt wurde. Insofern wird Biomethan abgegrenzt von synthetischem Methan, das durch physikalisch-chemisch technische Verfahren unter Verwendung von Katalysatoren hergestellt wird und auch zu natürlich entstandenem Methan, das beispielsweise aus natürlichen Ergasvorkommen gefördert wird. Von dem Methan biogenen Ursprungs, das in einer konventionellen Biogasanlage als Anteil des biomethanhaltigen Gases gebildet wird, unterscheidet sich das erfindungsgemäße Biomethan dadurch, dass die Ausgangsstoffe für die Biomethanbildung Wasserstoff und Kohlendioxid sind und nicht ein organisches Gärsubstrat. Ein "biomethanhaltiges Gas" ist jedes Gas, das einen messbaren Anteil an Biomethan aufweist.

Die erfindungsgemäße Energieversorgungseinheit umfasst eine Elektrolysevorrichtung zur Erzeugung von Wasserstoff und Sauerstoff aus Wasser unter Verwendung zumindest eines Teils des von der Windkraftanlage erzeugten elektrischen Stroms. In einer bevorzugten Ausführungsform wird insbesondere der billige Überschussstrom zum Betreiben der Elektrolysevorrichtung verwendet. Ein zur Windkraftanlage gehörender Netzregler steuert und regelt die Verteilung des aus Windkraft vor Ort produzierten elektrischen Stroms sowie die gegebenenfalls aus dem Stromnetz zur Verfügung stehende billige elektrische Energie aus Überschussstrom.

Die Elektrolyse wird bevorzugt nicht kontinuierlich betrieben, sondern diskontinuierlich. In einer Ausführungsform kann zwar kontinuierlich ein gewisser Grundstrom zur Verfügung gestellt werden, so dass die Elektrolysevorrichtung nicht vollkommen abgeschaltet wird, sondern der Prozess auf einem niedrigen Niveau aufrecht erhalten bleibt, aber die elektrolytisch produzierte Menge an Wasserstoff schwankt gekoppelt an die Verfügbarkeit an günstigem Strom erheblich.

In einer Ausführungsform wird die Elektrolysevorrichtung als externer Elektrolyseur zur Verfügung gestellt. Dieser kann ortsnah im Bereich der Windkraftanlage installiert sein, bevorzugt aber direkt neben, an oder in dem Turm der Windkraftanlage selber. Es können kommerziell erhältliche Elektrolyseure zur Elektrolyse von Wasser verwendet werden, insbesondere Systeme, die nach den Prinzipien der alkalischen Elektrolyse, der PEM-Elektrolyse (PEM, polymer electrolyte membrane) oder der AEM-Elekrolyse (alkaline solid polymer electrolyte membrane; ACTA) arbeiten.

In einer bevorzugten Ausführungsform wird die bei der Elektrolyse erzeugte Abwärme genutzt, um den Bioreaktor zu heizen. In einer bevorzugten Ausführungsform wird die Abwärme über einen Wärmetauscher dem Heizmedium für den Bioreaktor zugeführt. Durch die räumliche Nähe von Elektrolyseur und Bioreaktor zur Methanisierung im Turm der Windkraftanlage ergeben sich weitere vorteilhafte Aspekte. Der produzierte Wasserstoff fällt bei einigen Elektrolyseurtypen mit einem hohen Druck (bis zu 60 bar) an. Der Wasserstoff kann anschließend mit erhöhtem Druck in den Bioreaktor zur Methanbildung eingeleitet werden, um so eine höhere Löslichkeit des Wasserstoffs zu erreichen.

In der Regel ist der bei der Elektrolyse entstehende Wasserstoff nicht vollkommen trocken, sondern enthält Wasserdampf. Für technische Anwendungen muss in der Regel dem Wasserstoff durch Entfeuchtung über einen Kondensator Wasser entzogen werden. Wird der Wasserstoff direkt in den Bioreaktor eingeleitet, entfällt dieser Verfahrensschritt, da der Wassergehalt in dem biologischen Methanisierungsreaktor kein Problem darstellt. In einer bevorzugten Ausführungsform wird der in der externen Elektrolyseeinheit produzierte Wasserstoff direkt über eine ventilgesteuerte Zufuhrvorrichtung in den Bioreaktor zur Methanisierung eingebracht, insbesondere bevorzugt in Bodennähe des Reaktors.

In Zeiten, in denen mehr Wasserstoff produziert wird als in den Bioreaktor eingeleitet werden kann bzw. dort von den methanogenen Bakterien umgesetzt werden kann, wird gemäß einer bevorzugten Ausführungsform der Wasserstoff zwischengespeichert. Als Zwischenspeicher kann ein geeigneter Behälter dienen oder es kann Wasserstoff in entsprechende Druckflaschensysteme abgefüllt oder in Metallhydridspeichern zwischengespeichert werden. Bevorzugt wird der zwischengespeicherte Wasserstoff in Zeiten, in denen kein billiger Überschussstrom zur Verfügung steht, in den Bioreaktor eingeleitet.

Der ebenfalls bei der Elektrolyse in einem externen Elektrolyseur produzierte Sauerstoff wird gemäß einer bevorzugten Ausführungsform in die Umgebung abgelassen. In einer weiteren Ausführungsform wird der produzierte Sauerstoff in geeignete Behälter, z.B. Sauerstoffdruckflaschen abgefüllt und kann kommerziell verwertet werden, beispielsweise zum Schweißen.

Gemäß einer weiteren bevorzugten Ausführungsform erfolgt die Elektrolyse von Wasser in situ, also direkt im Bioreaktor. Der für die Methanisierung benötigte Wasserstoff wird direkt an Elektroden im Bioreaktor durch Wasserelektrolyse gebildet und steht den hydrogenotrophen methanogenen Bakterien dann direkt im Reaktor zur Verfügung. Die Wasserstoffbildung erfolgt an der Kathode. Aus dem Stand der Technik ist bekannt, dass sich geeignete Bakterien direkt an der Kathode anlagern können. Die Sauerstoffbildung erfolgt an der Anode.

Das erfindungsgemäße Energieversorgungssystem umfasst eine Zufuhrvorrichtung für Kohlendioxid. Die hydrogenotrophen Methanbakterien benutzen Kohlendioxid als Kohlenstoffquelle, um dieses zusammen mit Wasserstoff in Methan umzusetzen. Als Kohlendioxidquelle eignet sich beispielsweise CO₂ aus anaerober Fermentation, z.B. aus Biogasanlagen oder Kläranlagen, CO₂, das als Nebenprodukt bei Brauereien oder Brennereien anfällt, CO₂ aus industriellen Abgasen, z.B. aus Kraftwerken, Stahl- oder Zementwerken, CO₂ aus Verbrennungsabgasen, CO₂ aus der Vergasung von Biomasse, Abfall oder anderen kohlenstoffhaltigen Substanzen.

Die CO₂-haltigen Gase können weiter angereichert werden, so dass bevorzugt ein kohlendioxidhaltiges Gas mit einem CO₂-Gehalt von mehr als 50 %, besonders bevorzugt einem CO₂-Gehalt von mehr als 75 %, insbesondere bevorzugt einem CO₂-Gehalt von mehr als 90 % zur biologischen Methanisierung eingesetzt wird. Hochkonzentriertes Kohlendioxid entsteht beispielsweise als Nebenprodukt bei der Aufbereitung biomethanhaltiger Gase auf Erdgasqualität. Geringe Konzentrationen an Schwefelwasserstoff, die ebenfalls häufig als Nebenprodukt bei der Gasaufbereitung anfallen, stören nicht, sondern dienen in dem Bioreaktor in Anwesenheit von Methanbakterien als Reduktionsmittel.

In einer bevorzugten Ausführungsform kann das CO₂ auch durch Abtrennung aus der Luft gewonnen werden. Geeignet sind hierbei insbesondere Verfahren, die CO₂ chemisch an einen Absorber binden (insbesondere Absorbermaterialien auf Aminbasis) oder physikalisch an einen porösen Adsorber binden (z.B. Zeolithe, Aktivkohle, Kieselgel), von dem es später in angereicherter Form wieder desorbiert werden kann. Zwischen Absorption und Adsorption wird im Weiteren nicht unterschieden. Geeignete Beispiele sind in der WO 2008/021700, der WO2008/131132 und der WO2010/091831 dargestellt. Synergieeffekte ergeben sich in der Energieversorgungseinheit gemäß der vorliegenden Erfindung dadurch, dass bei diesen Verfahren der CO₂-Gewinnung aus Luft die vor Ort durch die Wasserelektrolyse anfallende Niedertemperaturabwärme genutzt werden kann, um gebundenes CO₂ wieder freizusetzen. Vorteilhaft ist auch, dass man bei dieser Ausführungform unabhängig von lokal zur Verfügung stehenden anderweitigen CO₂-Quellen arbeiten kann. Diese Verfahren eignen sich insbesondere für die CO₂-Bereitstellung bei "Off-shore"-Windanlagen oder auch bei "On-shore"-Windanlagen, die weit entfernt von der Zivilisation lokalisiert sind, da hier Kohlendioxid aus anderen Quellen schwerer verfügbar ist.

Im Falle der Nutzung von Verfahren zur CO₂-Abtrennung aus der Luft ergibt sich im Zusammenhang mit der Energieversorgungseinheit gemäß der vorliegenden Erfindung ein weiterer Synergieeffekt. Die oben beschriebenen Verfahren zur CO₂-Abtrennung aus der Luft benötigen einen Luftstrom durch das Absorbermaterial, um das darin enthaltene CO₂ zu binden. Je stärker dieser Luftstrom ist, also je mehr Luft durch den Absorber hindurchtritt, desto mehr CO₂ kann gebunden werden. Da Windkraftanlagen *per se* in Gebieten gebaut werden, in denen ein hohes Windaufkommen herrscht, ist die Kombination von CO₂-Gewinnung aus Luft vor Ort und H₂-Synthese vor Ort mit einer anschließenden Methanisierung vor Ort in der Energieversorgungseinheit gemäß der vorliegenden Erfindung besonders effizient.

Gemäß einer weiteren bevorzugten Ausführungsform kann die Effizienz der CO₂-Absorption aus Luft noch erhöht werden. Wird ein entsprechendes Absorbermodul für die CO₂-Absorption aus Luft ebenfalls in den Turm der Windkraftanlage eingebaut, kann der hohe schlanke Turm der Windkraftanlage wie ein Schornstein wirken, wenn oben und unten Ein- und Austrittsöffnungen für Luft vorhanden sind. Besonders bevorzugt ist am unteren Ende des Turms eine Wärmequelle vorhanden, so dass die eintretende Luft angewärmt wird. Bei einem derartigen Aufbau tritt ein Kamineffekt auf, der zu einer größeren Strömungsgeschwindigkeit der Luft durch das im Turm befindliche CO₂-Absorbermodul und damit einer effizienteren CO₂-Bindung führt. Als Wärmequelle für den Kamineffekt kann die Abwärme des Elektrolyseurs oder eine andere Heizung (z.B. Heizung mit Strom aus Windkraftanlage) dienen.

In einer weiteren Ausführungsform kann das CO₂ durch Abtrennung aus Wasser, insbesondere aus Meerwasser gewonnen werden.

Kohlendioxid oder kohlendioxidhaltiges Gas wird bevorzugt über eine ventilgesteuerte Leitung dem Bioreaktor in der Windkraftanlage zugeführt. In einer weiter bevorzugten Ausführungsform wird Kohlendioxid in Bodennähe des Bioreaktors zugeführt. In einer bevorzugten Ausführungsform wird das Kohlendioxid möglichst fein verteilt zugeführt, beispielsweise über entsprechende Fritten, feinporige Verteilerplatten, netzartige Verteilergewebe oder entsprechende Gaslanzen.

Das erfindungsgemäße Energieversorgungssystem umfasst einen Auslass für das in dem Bioreaktor entstehende biomethanhaltige Gas. Das biomethanhaltige Gas kann neben Methan auch Anteile von H₂ und CO₂ sowie kleinere Konzentrationen an beispielsweise H₂S, O₂, N₂ und Wasser enthalten.

Der Auslass für das biomethanhaltige Gas befindet sich bevorzugt im oberen Bereich des Bioreaktors. Er ist durch ein Ventil absperrbar. Das Abführsystem für das biomethanhaltige Gas weist bevorzugt Messeinrichtungen für die Gasqualität, insbesondere Messeinrichtungen für CH₄, H₂, CO₂, O₂ und H₂S auf sowie Messeinrichtungen für den Gasstrom. Diese Messeinrichtungen sind Teil eines Regelungs- und Steuerungssystems, das abhängig von der Gaszusammensetzung und Gasmenge sämtliche Gaszu- und -abflüsse des Bioreaktors steuert und regelt. Ist beispielsweise der Anteil an H₂ oder CO₂ in dem entstehenden biomethanhaltigen Gas zu hoch (z.B. mehr als 5 % Restwasserstoff), kann das biomethanhaltige Gas in den Bioreaktor rezirkuliert werden, so dass nicht umgesetztes H₂ und/oder CO₂ von den Methanbakterien in CH₄ umgewandelt wird. Besonders bevorzugt ist es, das biomethanhaltige Gas im oberen Teil des Bioreaktors zu entnehmen und am unteren Ende des Bioreaktors rückzuführen.

Das biomethanhaltige Gas kann über eine Gasleitung direkt in eine Gasverwertungseinrichtung geleitet werden oder es wird alternativ zuvor in einer Gasaufbereitungseinrichtung aufbereitet, wobei der Biomethangehalt erhöht wird und andere Anteile wie H₂, CO₂, H₂S, O₂, N₂ und Wasser abgetrennt werden. Methoden zur Gasaufbereitung sind im Stand der Technik hinreichend bekannt. Das biomethanhaltige Gas wird anschließend bevorzugt einer Verwertungseinrichtung zugeführt. Diese kann beispielsweise ein Blockheizkraftwerk zur Strom- und Wärmeproduktion, ein Gasbrenner oder eine Mikrogasturbine sein. Bevorzugt wird das Biomethan direkt in eine Erdgasleitung eingespeist, in einem Gasspeicher zwischengespeichert oder als Bioerdgas für Kraftfahrzeuge zur Verfügung gestellt.

Die vorliegende Erfindung umfasst auch ein Verfahren zum Betrieb einer der oben näher erläuterten Energieversorgungseinheit. Das Verfahren umfasst die Schritte Betrieb einer Windkraftanlage zur Erzeugung von elektrischem Strom aus Wind, Betrieb einer Elektrolysevorrichtung zur Erzeugung von Wasserstoff und Sauerstoff aus Wasser unter Verwendung zumindest eines Teils des von der Windkraftanlage erzeugten Überschussstroms, Betrieb eines in dem Turm der Windkraftanlage eingebauten Bioreaktors zur Herstellung von Methan aus Wasserstoff und Kohlendioxid unter Einsatz von methanogenen Bakterien, Zufuhr von Wasserstoff in den Bioreaktor und/oder Bildung von Wasserstoff in dem Bioreaktor, Zufuhr von Kohlendioxid in den Bioreaktor und Ablassen des im Bioreaktor entstehenden biomethanhaltigen Gases durch einen in dem Bioreaktor vorgesehenen Auslass.

Die besonderen Vorteile dieses Verfahrens, die bevorzugten Ausführungsformen dieses Verfahrens und die besonderen Vorteile solcher bevorzugten Ausführungsformen dieses Verfahrens zum Betrieb einer Energieversorgungseinheit entsprechen den Vorteilen bzw. den bevorzugten Ausführungsformen der erfindungsgemäßen Energieversorgungseinheit und wurden in diesem Zusammenhang bereits näher erläutert.

### Kurze Beschreibung der Zeichnungen

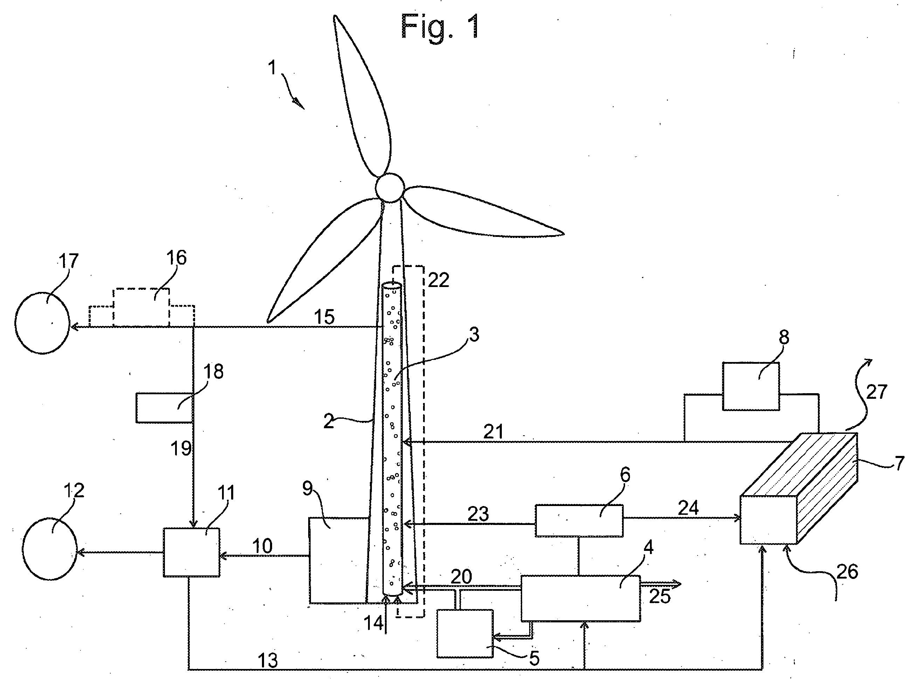

Zur Illustration der Erfindung und zur Verdeutlichung ihrer Vorzüge wird nachfolgend ein Ausführungsbeispiel angegeben. Das Ausführungsbeispiel wird im Zusammenhang mit Figur 1 näher erläutert. Es versteht sich von selbst, dass die im Zusammenhang mit den Ausführungsbeispielen gemachten Angaben die Erfindung nicht beschränken sollen.

Figur 1 zeigt eine schematische Darstellung einer Energieversorgungseinheit gemäß der vorliegenden Erfindung mit einem im Turm der Windkraftanlage eingebauten Bioreaktor, einem Elektrolyseur zur Wasserstoffproduktion und einer Kohlendioxid-Gewinnung aus Luft.

### Wege zur Ausführung der Erfindung

Figur 1 zeigt schematisch eine mögliche Ausführungsform einer Energieversorgungseinheit gemäß der vorliegenden Erfindung. Dargestellt sind die einzelnen Komponenten der Energieversorgungseinheit und die entsprechenden Stoffströme zwischen den einzelnen Komponenten. Die Energieversorgungseinheit weist eine Windkraftanlage 1 auf, in deren Turm 2 ein Bioreaktor 3 zur biologischen Methanisierung eingebaut ist. Weiterhin besitzt die Energieversorgungseinheit einen Elektrolyseur 4 zur Erzeugung von Wasserstoff und Sauerstoff. Der Wasserstoff kann gegebenenfalls in einem Wasserstoffzwischenspeicher 5 gespeichert werden. Die im Elektrolyseur 4 bei der Wasserelektrolyse produzierte Abwärme wird über einen Wärmetauscher 6 weiteren Anwendungen zur Verfügung gestellt.

Die Energieversorgungeinheit umfasst außerdem eine Kohlendioxidquelle, die in der gezeigten Ausführungsform aus dem CO₂-Bindemodul 7 besteht. Durch das CO₂-Bindemodul 7 kann CO₂ aus der Luft gebunden und anschließend wieder desorbiert und damit zur Verfügung gestellt werden. Das gebundene und wieder desorbierte CO₂ kann in einem CO₂-Zwischenspeicher 8 zwischengespeichert werden.

Die Windkraftanlage 1 besitzt außerdem einen Windstromgenerator 9, der Windstrom 10 über den Netzregler 11 entweder in das Stromnetz 12 einspeist oder ausgehend vom Netzregler 11 für eine Verwendung innerhalb der Energieversorgungseinheit über die interne Stromzufuhr 13 als "Überschussstrom" zur Verfügung stellt. Die interne Stromzufuhr 13 versorgt sowohl den Elektrolyseur 4 als auch die Kohlendioxidquelle mit dem CO₂-Bindemodul 7 mit entsprechendem Überschussstrom.

Der Bioreaktor 3 verfügt über eine Medienzufuhr 14 für Kulturmedien sowie über eine Medienabfuhr (nicht dargestellt). Das entstehende biomethanhaltige Gas wird über eine entsprechende Gasleitung 15 aus dem Bioreaktor 3 abgeführt. Das biomethanhaltige Gas kann gegebenenfalls über eine Gasaufbereitungseinrichtuhg 16 aufbereitet und anschließend in das Gasnetz oder in einen Gasspeicher 17 eingeleitet werden. Alternativ kann das biomethanhaltige Gas in einem Blockheizkraftwerk (BHKW) 18 verwertet werden. Hierbei entsteht Strom, der über die Stromleitung 19 und den Netzregler 11 entweder ins Stromnetz eingespeist wird oder über die interne Stromzufuhr 13 einer Verwertung innerhalb der Energieversorgungseinheit zugeführt wird. Zusätzlich entsteht bei der Verbrennung des biomethanhaltigen Gases im BHKW 18 Wärme, die entweder extern verwertet oder über einen Wärmetauscher analog zu dem Wärmetauscher 6 für Anwendungen innerhalb der Energieversorgungseinheit zur Verfügung gestellt werden kann (nicht dargestellt).

Der Bioreaktor 3 besitzt außerdem eine Zufuhrleitung für Wasserstoff 20 aus dem Elektrolyseur 4 oder dem H₂-Zwischenspeicher 5. Weiterhin umfasst der Bioreaktor 3 eine CO₂-Zufuhrleitung 21, durch die CO₂ direkt aus dem CO₂-Bindemodul 7 oder aus dem CO₂-Zwischenspeicher 8 zugeführt werden kann. Der Bioreaktor 3 weist gegebenenfalls eine Rezirkulationsleitung 22 für Kulturmedium und/oder im Bioreaktor gebildetes biomethanhaltiges Gas auf.

Die im Elektrolyseur 4 entstehende Abwärme wird über den Wärmetauscher 6 sowohl dem Bioreaktor 3 als Fermenterheizung 23 als auch dem CO₂-Bindemodul 7 als Wärme 24 für die CO₂-Desorption zur Verfügung gestellt. Der bei der Wasserelektrolyse im Elektrolyseur 4 gebildete Sauerstoff wird über eine O₂-Abfuhrleitung 25 abgeführt. Dem CO₂-Bindemodul 7 der Kohlendioxidquelle wird ein CO₂-haltiger Luftstrom 26 zugeführt. Der CO₂-abgereicherte Luftstrom 27 wird auf der der Luftzufuhr gegenüberliegenden Seite des CO₂-Bindemoduls 7 aus diesem abgeführt. Die Energieversorgungseinheit weist außerdem ein Mess-, Regel- und Steuerungssystem für die Regelung und Steuerung der Stoffströme auf. Dieses ist in der Figur 1 mit Ausnahme des Netzreglers 11 nicht dargestellt.

Eine Energieversorgungseinheit, wie sie schematisch in der Figur 1 dargestellt ist, kann exemplarisch in der nachfolgend erläuterten Dimensionierung sowie mit den nachfolgend beschriebenen Stoffströmen und Energieflüssen betrieben werden. Die Energieversorgungseinheit weist eine an Land gängige Dimensionierung für eine Windkraftanlage mit einem Rotordurchmesser von 82 m und einem Betonturm von 97 m Höhe auf. Eine Windkraftanlage, beispielsweise vom Typ Enercon E-82, liefert eine Nennleistung von 2300 kW. Der in den Turm 2 der Windkraftanlage 1 eingebaute Bioreaktor zur Methanisierung 3 weist ein Reaktorvolumen von 6 m³ auf. Bei einer zylinderförmigen Reaktorgeometrie weist der Bioreaktor bei einer Höhe von 10 m beispielsweise einen Durchmesser von 87 cm auf, bei einer Höhe von 80 m einen Durchmesser von 31 cm.

Der mit methanogenen Mikroorganismen der Art *Methanothermobacter thermautotrophicus* bei 65 °C betriebene Bioreaktor weist eine Methanbildungsrate von 100 m³ CH₄ pro m³ Reaktorvolumen pro Tag auf. Bei einem Reaktorvolumen von 6 m³ und einem vollständigen Umsatz der zugeführten Ausgangsgase müssen dem Reaktor pro Stunde 100 m³ H₂ und 25 m³ CO₂ zugeführt werden. Es werden pro Tag 600 m³ Biomethan mit einem Brennwert von ca. 6 MWh produziert.

Der Wasserstoff wird mit einem vor Ort installierten Elektrolyseur 4 produziert. Für die Herstellung von 1 m³ Wasserstoff durch Wasserelektrolyse verbraucht der Elektrolyseur 4 etwa 5 kWh an Strom. Würde der Elektrolyseur 4 kontinuierlich betrieben, müssten ca. 22 % der Leistung der Windkraftanlage für die Elektrolyse aufgewendet werden. Für eine diskontinuierliche Elektrolyse, angepasst an die Verfügbarkeit von günstigem Strom, der nicht direkt ins Netz eingespeist werden soll, muss die Windkraftanlage ca. 5 h pro Tag den kompletten erzeugten Strom an den Elektrolyseur 4 liefern, um die Menge von 2400 m² H₂ pro Tag zu erzeugen, die dann kurzfristig in einem Wasserstoffzwischenspeicher 5 gelagert werden muss.

Bei der Wasserelektrolyse fallen ca. 20 % der eingesetzten Energie als Abwärme an, also ca. 100 kWh Wärme pro 100 m³ erzeugten Wasserstoff. Diese Abwärme wird über einen Wärmetauscher 6 genutzt, um den Bioreaktor 3 zu heizen. Gleichzeitig speichert ein gut isolierter Bioreaktor 3 die Wärme auch in einem gewissen Maß und muss nicht kontinuierlich beheizt werden. Die methanogenen Mikroorganismen arbeiten zudem über einen größeren Temperaturbereich (ca. 40 °C bis 75 °C) und müssen nicht kontinuierlich exakt auf ihrer Optimaltemperatur gehalten werden.

Das für die Methanogenese erforderliche CO₂ wird in der beispielhaften Energieversorgungseinheit aus der Atmosphäre gewonnen, indem es in einem CO₂-Bindemodul 7 auf einem speziellen Adsorbermaterial adsorbiert und anschließend in konzentrierter Form desorbiert wird. Mit dem in der EP 2 266 680 beschriebenen Adsorbermaterial gelingt es, bei einer Windgeschwindigkeit von 10 m/s auf 1 m² Adsorberfläche pro Stunde 7 m³ CO₂ zu fixieren. Mit einer installierten Adsorberfläche von 3,5 m² könnte so eine Kohlendioxidmenge von 25 m² pro Stunde zur Verfügung gestellt werden.

Die Adsorption von Kohlendioxid erfolgt über den vorhandenen Luftstrom 26 und wird durch Wind gefördert, so dass dieses Verfahren zur Kohlendioxidgewinnung sehr gut mit der erfindungsgemäßen Energieversorgungseinheit korreliert, bei dem der Bioreaktor 3 im Turm der Windkraftanlage eingebaut ist. Für die Desorption des Kohlendioxids ist eine erhöhte Temperatur, vorzugsweise eine Temperatur von 80 °C bis 100 °C, erforderlich. Für die Produktion von 25 m³ CO₂ liegt der Energieverbrauch nach dem beschriebenen Verfahren bei etwa 50 kWh. Davon werden etwa 95 % als Wärme benötigt und nur 5 % als elektrische Energie in Form von Strom. Die 47,5 kWh in Form von Wärmeenergie können über den Wärmetauscher 6 aus dem Elektrolyseur 4 entnommen werden, die entsprechend 100 kWh Abwärme pro 100 m³ erzeugten Wasserstoff produziert. Die Elektrolyseabwärme kann also direkt vor Ort über den Wärmetauscher 6 sowohl für die Fermenterheizung 23 als auch für die Kohlendioxidproduktion genutzt werden, was die Energieeffizienz für die Methangewinnung verbessert.

Die erforderlichen 2,5 kWh an elektrischer Energie für eine Gewinnung von 25 m³ CO₂ aus der Umgebungsluft werden ebenfalls in Form von günstigem Überschussstrom zur Verfügung gestellt. Ein Kohlendioxidzwischenspeicher 8 vor Ort sorgt dafür, dass CO₂ kurzfristig zwischengespeichert werden kann. Ein entsprechendes Regelungs- und Steuerungssystem gewährleistet, dass sowohl für den Elektrolyseur 4 mit Wärmetauscher 6 als auch für die Kohlendioxidquelle jeweils günstiger Überschussstrom aus der Windkraftanlage 1 bereitgestellt wird. Außerdem regelt und steuert das System die erforderlichen Gas- und Flüssigkeitsströme in der Energieversorgungseinheit und insbesondere in-den Bioreaktor hinein und vom Bioreaktor weg.

Bei einer vollständigen Umsetzung des zur Verfügung gestellten Wasserstoffs und Kohlendioxids durch die methanogenen Mikroorganismen zu Biomethan entstehen in der beispielhaften Energieversorgungseinheit pro Tag 600 m³ Biomethan mit einem Energiegehalt (Brennwert) von ca. 6 MWh. Für die Elektrolyse zur Erzeugung der entsprechenden 2400 m³ Wasserstoff werden 12 MWh an Strom verbraucht, für die Bereitstellung der 600 m³ Kohlendioxid 60 kWh an Strom, so dass sich bei entsprechender Abwärmenutzung ein Wirkungsgrad von fast 50 % für die biologische Methanisierung ergibt, wenn das entstandene Biomethan direkt ins Gasnetz eingespeist oder vor Ort weiterverwertet werden kann.

Vor dem Hintergrund, dass die Windkraftanlage alternativ in bestimmten Zeiten gar keinen Strom produziert, weil er nicht ins Netz eingespeist werden kann, erscheint dies eine wirtschaftlich rentable Lösung für die aktuellen Probleme bei der Windstromerzeugung und -verwertung.

## Patentansprüche

1. Energieversorgungseinheit, aufweisend
- eine Windkraftanlage (1) zur Erzeugung von elektrischem Strom aus Wind,
- eine Elektrolysevorrichtung (4) zur Erzeugung von Wasserstoff und Sauerstoff aus Wasser unter Verwendung zumindest eines Teils des von der Windkraftanlage (1) erzeugten elektrischen Stroms,
- einen Bioreaktor (3) zur Herstellung von Methan aus Wasserstoff und Kohlendioxid unter Einsatz von methanogenen Bakterien,
- eine Zufuhrvorrichtung für die Zufuhr von Kohlendioxid in den Bioreaktor (3) und
- einen in dem Bioreaktor (3) vorgesehenen Auslass für das im Bioreaktor (3) entstehende biomethanhaltige Gas,
**dadurch gekennzeichnet, dass**
der Bioreaktor (3) in dem Turm (2) der Windkraftanlage (1) eingebaut vorliegt und der Bioreaktor (3) eine Höhe von mehr als 5 m aufweist.

2. Energieversorgungseinheit nach Anspruch 1, **dadurch gekennzeichnet, dass** der Bioreaktor (3) eine Höhe von mehr als 10 m, insbesondere von mehr als 20 m und besonders bevorzugt von mehr als 100 m aufweist.

3. Energieversorgungseinheit nach Anspruch 2, **dadurch gekennzeichnet, dass** das Verhältnis von Höhe zu Durchmesser des Bioreaktors (3) mehr als 1:1, bevorzugt mehr als 5:1, besonders bevorzugt mehr als 10:1 beträgt.

4. Energieversorgungseinheit nach einem der vorhergehenden Ansprüche, **dadurch gekennzeichnet, dass** der Bioreaktor (3) eine Zufuhr- und/oder Abfuhr-Vorrichtung für die Zufuhr und/oder Abfuhr eines Kulturmediums umfasst.

5. Energieversorgungseinheit nach einem der vorhergehenden Ansprüche, **dadurch gekennzeichnet, dass** es sich bei der Zufuhrvorrichtung für die Zufuhr von Kohlendioxid um eine ventilgesteuerte Leitung in Bodennähe des Bioreaktors (3) handelt.

6. Energieversorgungeinheit nach einem der vorhergehenden Ansprüche, **dadurch gekennzeichnet, dass** ein Modul (7) zur Gewinnung des für die Methanisierung verwendeten Kohlendioxids durch Kohlendioxidabsorption oder -adsorption aus Luft vorgesehen ist, wobei bevorzugt das für die Kohlendioxidabsorption oder - adsorption aus Luft verwendete Modul (7) in den Turm (2) der Windkraftanlage (1) eingebaut vorliegt und der Turm (2) der Windkraftanlage (1) zur Erzeugung eines Kamineffekts Lufteintritts- und -austrittsvorrichtungen sowie eine Heizvorrichtung zur Erwärmung der Luft aufweist.

7. Energieversorgungseinheit nach einem der vorhergehenden Ansprüche, **dadurch gekennzeichnet, dass** es sich bei der Elektrolysevorrichtung (4) zur Erzeugung von Wasserstoff und Sauerstoff aus Wasser um einen Elektrolyseur handelt, wobei der Elektrolyseur (4) bevorzugt benachbart zu dem Turm (2) der Windkraftanlage (1) oder im Turm (2) der Windkraftanlage (1) oder in den Bioreaktor (3) integriert vorgesehen ist.

8. Energieversorgungseinheit nach einem der vorhergehenden Ansprüche, **dadurch gekennzeichnet, dass** der Bioreaktor (3) eine Rückführ-Vorrichtung zur Rückführung des entstehenden biomethanhaltigen Gases in den Bioreaktor (3) aufweist, wobei ein Einlass für das rückgeführte biomethanhaltige Gas sich bevorzugt in Bodennähe des Bioreaktors (3) befindet.

9. Energieversorgungseinheit nach einem der vorhergehenden Ansprüche, **dadurch gekennzeichnet, dass** es sich bei den methanogenen Bakterien um mesophile und/oder um thermophile Bakterien handelt, wobei es sich bei den methanogenen Bakterien bevorzugt um hydrogenotrophe, methanbildende Bakterien handelt.

10. Energieversorgungseinheit nach Anspruch 9, **dadurch gekennzeichnet, dass** es sich bei den hydrogenotrophen, methanbildenden Bakterien der Ordnungen *Methanobacteriales, Methanomicrobiales, Methanococcales, Methanopyrales, Methanocellales, Methanosarcinales* oder deren Mischungen handelt, wobei es sich bei den hydrogenotrophen, methanbildenden Bakterien bevorzugt um Bakterien der Arten *Methanothermobacter thermautotrophicus, Methanothermobacter marburgensis, Methanothermobacter wolfeii, Methanococcus maripaludis, Methanococcus aeolicus, Methanococcus thermolithotrophicus, Methanobacterium formicicum* oder deren Mischungen handelt.

11. Energieversorgungseinheit nach einem der Ansprüche 9 oder 10, **dadurch gekennzeichnet, dass** zumindest eine Art von hydrogenotrophen, methanbildenden Bakterien anwesend ist und zumindest ein weitere Art methanbildender Bakterien, wobei es sich bei der weiteren Art methanbildender Bakterien bevorzugt um acetoclastische Methanbakterien, insbesondere um *Methanosarcina sp.* oder *Methanosaeta sp.* handelt.

12. Energieversorgungseinheit nach einem der Ansprüche 9 bis 11, **dadurch gekennzeichnet, dass** zumindest eine Art von hydrogenotrophen, methanbildenden Bakterien anwesend ist und zumindest ein Art hydrolytisch wirkender Bakterien.

13. Energieversorgungseinheit nach einem der vorhergehenden Ansprüche, **dadurch gekennzeichnet, dass** die Bakterien immobilisiert an einem Trägerstoff vorliegen.

14. Energieversorgungseinheit nach einem der vorhergehenden Ansprüche, **dadurch gekennzeichnet, dass** zusätzlich eine Verwertungseinheit zur Umwandlung des im Bioreaktor (3) entstehenden biomethanhaltigen Gases vorgesehen ist.

15. Verfahren zum Betrieb einer Energieversorgungseinheit nach einem der vorhergehenden Ansprüche umfassend die Schritte:
- Betrieb einer Windkraftanlage (1) zur Erzeugung von elektrischem Strom aus Wind,
- Betrieb einer Elektrolysevorrichtung (4) zur Erzeugung von Wasserstoff und Sauerstoff aus Wasser unter Verwendung zumindest eines Teils des von der Windkraftanlage (1) erzeugten elektrischen Stroms,
- Betrieb eines in dem Turm (2) der Windkraftanlage (1) eingebauten Bioreaktors (3) zur Herstellung von Methan aus Wasserstoff und Kohlendioxid unter Einsatz von methanogenen Bakterien, wobei der Bioreaktor (3) eine Höhe von mehr als 5 m aufweist,
- Zufuhr von Wasserstoff in den Bioreaktor (3) und/oder Bildung von Wasserstoff in dem Bioreaktor (3),
- Zufuhr von Kohlendioxid in den Bioreaktor (3),
- Ablassen des im Bioreaktor (3) entstehenden biomethanhaltigen Gases durch einen in dem Bioreaktor (3) vorgesehenen Auslass.

## Claims

1. Energy supply unit, comprising
- a wind power plant (1) for generating electric power from wind,
- an electrolysis device (4) for creating hydrogen and oxygen from water using at least part of the electric power generated by the wind power plant (1),
- a bioreactor (3) for producing methane from hydrogen and carbon dioxide using methanogenic bacteria,
- a supply device for supplying carbon dioxide to the bioreactor (3), and
- an outlet, provided in the bioreactor (3), for the biomethane-containing gas generated in the bioreactor (3),
**characterised in that**
the bioreactor (3) is installed in the tower (2) of the wind power plant (1), and the bioreactor (3) has a height of more than 5 m.

2. Energy supply unit according to claim 1, **characterised in that** the bioreactor (3) has a height of more than 10 m, particularly of more than 20 m and particularly preferably of more than 100 m.

3. Energy supply unit according to claim 2, **characterised in that** the ratio of height to diameter of the bioreactor (3) is more than 1:1, preferably more than 5:1, particularly preferably more than 10:1.

4. Energy supply unit according to any one of the preceding claims, **characterised in that** the bioreactor (3) comprises a supply and/or removal device for the supply and/or removal of a culture medium.

5. Energy supply unit according to any one of the preceding claims, **characterised in that** the supply device for supplying carbon dioxide is a valve-controlled line close to the bottom of the bioreactor (3).

6. Energy supply unit according to any one of the preceding claims, **characterised in that** a module (7) is provided for obtaining the carbon dioxide used for the methanation by means of carbon-dioxide absorption or adsorption from the air, wherein the module (7) used for the carbon-dioxide absorption or adsorption from the air is preferably installed in the tower (2) of the wind power plant (1) and the tower (2) of the wind power plant (1) has air-inlet and -outlet devices for creating a chimney effect as well as a heating device for heating the air.

7. Energy supply unit according to any one of the preceding claims, **characterised in that** the electrolysis device (4) for creating hydrogen and oxygen from water is an electrolyser, wherein the electrolyser (4) is preferably provided adjacently to the tower (2) of the wind power plant (1) or in the tower (2) of the wind power plant (1) or integrated into the bioreactor (3).

8. Energy supply unit according to any one of the preceding claims, **characterised in that** the bioreactor (3) comprises a return device for returning the generated biomethane-containing gas to the bioreactor (3), wherein an inlet for the returned biomethane-containing gas is preferably located close to the bottom of the bioreactor (3).

9. Energy supply unit according to any one of the preceding claims, **characterised in that** the methanogenic bacteria are mesophilic and/or thermophilic bacteria, wherein the methanogenic bacteria are preferably hydrogenotrophic, methane-forming bacteria.

10. Energy supply unit according to claim 9, **characterised in that** the hydrogenotrophic, methane-forming bacteria are of the orders *Methanobacteriales, Methanomicrobiales, Methanococcales, Methanopyrales, Methanocellales, Methanosarcinales* or mixtures thereof, wherein the hydrogenotrophic, methane-forming bacteria are preferably bacteria of the species *Methanothermobacter thermautotrophicus, Methanothermobacter marburgensis, Methanothermobacter wolfeii, Methanococcus maripaludis, Methanococcus aeolicus, Methanococcus thermolithotrophicus, Methanobacterium formicicum* or mixtures thereof.

11. Energy supply unit according to one of claims 9 or 10, **characterised in that** at least one species of hydrogenotrophic, methane-forming bacteria is present and at least one further species of methane-forming bacteria, wherein the further species of methane-forming bacteria are preferably acetoclastic methane bacteria, particularly *Methanosarcina sp.* or *Methanosaeta sp.*

12. Energy supply unit according to one of claims 9 to 11, **characterised in that** at least one species of hydrogenotrophic, methane-forming bacteria is present and at least one species of hydrolytically active bacteria.

13. Energy supply unit according to any one of the preceding claims, **characterised in that** the bacteria are present immobilised on a carrier material.

14. Energy supply unit according to any one of the preceding claims, **characterised in that** a utilisation unit for converting the biomethane-containing gas generated in the bioreactor (3) is additionally provided.

15. A method for operating an energy supply unit according to one of the preceding claims, comprising the steps:
- operating a wind power plant (1) for generating electric power from wind,
- operating an electrolysis device (4) for creating hydrogen and oxygen from water using at least part of the electric power generated by the wind power plant (1),
- operating a bioreactor (3), which is installed in the tower (2) of the wind power plant (1), for producing methane from hydrogen and carbon dioxide using methanogenic bacteria, wherein the bioreactor (3) has a height of more than 5 m,
- supplying hydrogen to the bioreactor (3) and/or forming hydrogen in the bioreactor (3),
- supplying carbon dioxide to the bioreactor (3),
- discharging the biomethane-containing gas generated in the bioreactor (3) by means of an outlet provided in the bioreactor (3).

## Revendications

1. Unité d'alimentation en énergie, présentant :
- une installation d'énergie éolienne (1) pour générer un courant électrique à partir du vent,
- un dispositif d'électrolyse (4) pour générer de l'hydrogène et de l'oxygène à partir de l'eau en utilisant au moins une partie du courant électrique généré par l'installation d'énergie éolienne (1),
- un bioréacteur (3) pour produire du méthane à partir de l'hydrogène et du dioxyde de carbone en utilisant des bactéries méthanogènes,
- un dispositif d'introduction pour introduire le dioxyde de carbone dans le bioréacteur (3) et
- une évacuation prévue dans le bioréacteur (3) pour le gaz contenant du biométhane généré dans le bioréacteur (3),
**caractérisée en ce que**
le bioréacteur (3) est monté dans la tour (2) de l'installation d'énergie éolienne (1) et le bioréacteur (3) présente une hauteur supérieure à 5 m.

2. Unité d'alimentation en énergie selon la revendication 1, **caractérisée en ce que** le bioréacteur (3) présente une hauteur de plus de 10 m, notamment de plus de 20 m et de manière particulièrement préférée de plus de 100 m.

3. Unité d'alimentation en énergie selon la revendication 2, **caractérisée en ce que** le rapport de la hauteur sur le diamètre du bioréacteur (3) s'élève à plus de 1 :1, de préférence plus de 5 :1 et de manière particulièrement préférée plus de 10 :1.

4. Unité d'alimentation en énergie selon une des revendications précédentes, **caractérisée en ce que** le bioréacteur (3) présente un dispositif d'alimentation et/ou d'évacuation pour l'alimentation/ou l'évacuation d'un milieu de culture.

5. Unité d'alimentation en énergie selon une des revendications précédentes, **caractérisée en ce que** le dispositif d'alimentation pour alimenter du dioxyde de carbone consiste en une conduite commandée par soupape à proximité du sol du bioréacteur (3).

6. Unité d'alimentation en énergie selon une des revendications précédentes, **caractérisée en ce que** un module (7) pour extraire le dioxyde de carbone utilisé pour la méthanisation est prévu par absorption ou adsorption de dioxyde de carbone à partir de l'air, dans laquelle de préférence le module (7) utilisé pour l'absorption ou l'adsorption de dioxyde de carbone à partir de l'air est monté dans la tour (2) de l'installation d'énergie éolienne (1) et la tour (2) de l'installation d'énergie éolienne (1) présente pour produire un effet de cheminée des dispositifs d'entrée et de sortie d'air ainsi qu'un dispositif de chauffage pour chauffer l'air.

7. Unité d'alimentation en énergie selon une des revendications précédentes, **caractérisée en ce que** le dispositif d'électrolyse (4) pour produire de l'hydrogène et de l'oxygène à partir de l'eau consiste en une électrolyseur, dans laquelle l'électrolyseur (4) est prévue de préférence à proximité de la tour (2) de l'installation d'énergie éolienne (1) ou est intégrée dans la tour (2) de l'installation d'énergie éolienne (1) ou dans le bioréacteur (3).

8. Unité d'alimentation en énergie selon une des revendications précédentes, **caractérisée en ce que** le bioréacteur (3) présente un dispositif de réintroduction pour réintroduire le gaz contenant du biométhane généré dans le bioréacteur (3), dans laquelle une admission pour le gaz contenant du biométhane réintroduit se trouve de préférence à proximité du sol du bioréacteur (3).

9. Unité d'alimentation en énergie selon une des revendications précédentes, caractérisé&e en ce que les bactéries méthanogènes consistent en bactéries mésophiles et/ou thermophiles, dans laquelle les bactéries méthanogènes consistent de préférence en bactéries formant du méthane, hydrogénotrophes.

10. Unité d'alimentation en énergie selon la revendication 9, **caractérisée en ce que** les bactéries formant du méthane, hydrogénotrophes consistent en bactéries de l'ordre *Methanobacteriales, Methanomicrobiales, Methanococcales, Methanopyrales, Methanocellales, Methanosarcinales* ou des mélanges de ces dernières, dans laquelle les bactéries formant du méthane, hydrogénotrophes consistent de préférence en bactéries des types *Methanothermobacter thermautotrophicus, Methanothermobacter marburgensis, Methanothermobacter wolfeii, Methanococcus maripaludis, Methanococcus aeolicus, Methanococcus thermolitrophicus, Methanobacterium formicicum* ou des mélanges de ces dernières.

11. Unité d'alimentation en énergie selon une des revendications 9 ou 10, **caractérisée en ce que** au moins un type des bactéries formant du méthane, hydrogénotrophes est présent et au moins un autre type de bactéries formant du méthane, dans lequel l'autre type de bactéries formant du méthane consiste de préférence en bactéries de méthane acétoclastique, notamment en *Methanosarcina* sp. ou *Methanosaeta* sp.

12. Unité d'alimentation en énergie selon une des revendications 9 à 11, **caractérisée en ce que** au moins un type des bactéries formant du méthane, hydrogénotrophes est présent et au moins un type de bactéries à action hydrolytique.

13. Unité d'alimentation en énergie selon une des revendications précédentes, **caractérisée en ce que** les bactéries sont immobilisées sur une matière support.

14. Unité d'alimentation en énergie selon une des revendications précédentes, **caractérisée en ce que** en outre une unité de valorisation pour convertir le gaz contenant du biométhane généré dans le bioréacteur (3) est présente.

15. Procédé de fonctionnement d'une unité d'alimentation en énergie selon une des revendications précédentes comprenant les étapes consistant à :
- faire fonctionner une installation d'énergie éolienne (1) pour générer un courant électrique à partir du vent,
- faire fonctionner un dispositif d'électrolyse (4) pour générer de l'hydrogène et de l'oxygène à partir de l'eau en utilisant au moins une partie du courant électrique généré par l'installation d'énergie éolienne (1),
- faire fonctionner un bioréacteur (3) monté dans la tour (2) de l'installation d'énergie éolienne (1) pour produire du méthane à partir de l'hydrogène et du dioxyde de carbone en utilisant des bactéries méthanogènes, dans lequel le bioréacteur (3) présent une hauteur de plus de 5 m,
- introduire de l'hydrogène dans le bioréacteur (3) et/ou former de l'hydrogène dans le bioréacteur (3),
- introduire du dioxyde de carbone dans le bioréacteur (3),
- évacuer le gaz contenant du biométhane généré dans le bioréacteur (3) par l'intermédiaire d'une évacuation prévue dans le bioréacteur (3).
